# EUROPEAN PATENT APPLICATION

(11) **EP 2 399 593 A1**
(43) Date of publication of application: **28.12.2011**
(21) Application number: 10305690.9
(22) Date of filing: 28.06.2010
(51) Int. Cl.: A61K 31/727, A61P 7/02

(54) **Semuloparin for use as an antithrombotic treatment in orthopaedic surgery with improved benefit-risk profile**

(71) Applicant: Aventis Pharma S.A., 92160 Antony (FR)
(72) Inventor: The designation of the inventor has not yet been filed
(74) Representative: Romanowski, Caroline

(57) **Abstract**

The invention relates to the use of semuloparin or a pharmaceutically acceptable salt thereof as an antithrombotic treatment in patients undergoing major orthopaedic surgery, wherein said use involves an improved benefit-risk profile, compared to a standard antithrombotic treatment.

## Description

The invention relates to the use of semuloparin or a pharmaceutically acceptable salt thereof as an antithrombotic treatment in patients undergoing major orthopaedic surgery, wherein said use involves an improved benefit-risk profile compared to a standard antithrombotic treatment.

Semuloparin, or AVE5026 (sanofi-aventis laboratory code) belongs to a new generation of hemisynthetic heparins. It is a new ultra-low molecular weight heparin, with an average molecular weight of 2000-3000 Daltons and a novel antithrombotic profile resulting from high anti-Factor Xa activity (∼ 160 U/mg) and residual anti-Factor IIa activity (∼ 2 U/mg), as described in Journal of Thrombosis and Haemostasis, 2009, vol. 7, 1143-1151. It is obtained by selective and controlled depolymerization of heparin, as described in the patent application WO 2004/033503 and in J. Thromb Haemost. (ibid). Semuloparin, in the form of its sodium salt, is in clinical development for venous thromboembolism prevention.

At the present time, low-molecular-weight heparins (LMWHs), the synthetic pentasaccharide fondaparinux or dose-adjusted anti-vitamin K are the primary treatments for the prevention of venous thromboembolic diseases.

Patients undergoing surgery are at substantial risk of postoperative venous thromboembolism (hereafter "VTE"). In addition, many hospitalized patients have additional risk factors for VTE. In order to avoid postoperative venous thromboembolic complication, guidelines in thrombosis (ACCP guidelines) recommend the use of antithrombotic drugs for certain categories of surgical patients. Amongst these drugs, the LMWH enoxaparin is the pharmacological VTE prevention agent with the highest clinical documentation in surgical populations and with the largest clinical use in this setting. Enoxaparin has an average molecular weight of 3800-5000 Daltons, an anti-Factor Xa activity comprised between 90 and 125 IU/mg and an anti-Factor IIa activity of 20-35 IU/mg.

These therapies are effective, but the antithrombotic properties of such drugs are accompanied by a risk of haemorrhage.

The Applicant has now found that a product outside of the LMWH class, namely the ultra-low molecular weight heparin (ULMWH) semuloparin, displays an advantageous benefit-risk profile when used in orthopaedic surgery.

Therefore, the subject-matter of the invention is semuloparin or a pharmaceutically acceptable salt thereof for use as an antithrombotic treatment in patients undergoing major orthopaedic surgery, wherein said use involves an improved benefit-risk profile for the prevention of venous thromboembolism and death, compared to a standard antithrombotic treatment.

The term semuloparin, in the framework of the instant invention, encompasses any pharmaceutically acceptable salt thereof, in particular its sodium salt.

According to the instant invention, the terms below have the following meanings:
- "treatment" refers to the administration of a therapy to an individual who is considered as being at risk for a thromboembolic pathology, in particular venous thromboembolism (VTE), such as deep vein thrombosis, which may lead to pulmonary embolism. It shall therefore be understood that the term "treatment" as used in the instant invention refers to a prophylactic treatment of venous thromboembolism;
- "patient" refers to a patient being at risk of VTE and for whom prophylaxis of venous thromboembolic events is indicated;
- "benefit-risk profile", in the framework of the instant invention, refers to an improved efficacy with similar safety, compared to a standard antithrombotic treatment. "Benefit-risk profile" can also be understood as "benefit-to-risk ratio" in the framework of the instant invention.

According to the instant invention, the efficacy of the antithrombotic treatment is assessed by measuring the occurrence of venous thrombo-embolic events, including deep vein thrombosis, non-fatal pulmonary embolism and death, while the safety of the antithrombotic treatment is measured by the occurrence of bleeding events, more particularly clinically relevant bleedings or major bleedings.

The term "clinically relevant bleedings" designates bleeding events considered as:
- major bleedings, or
- clinically relevant non-major bleedings.

A major bleeding designates any overt bleeding (visually observed, including by ultra-sound, lavage or abdominal computerized tomography scan), associated with at least one of the following:
- Fatal bleeding (major contributor to death);
- symptomatic bleeding in a critical area or organ, such as intracranial, intraspinal, intraocular, retroperitoneal, intraarticular or pericardial, or intramuscular leading to a compartment syndrome;
- Bleeding causing a post-operative fall of hemoglobin level of 2 g/dL (1.24 mmol/L) or more compared to the first post-operative hemoglobin value or the most recent pre-bleeding hemoglobin value, or leading to a post-operative transfusion of two or more units of whole blood or red cells;
- Bleeding leading to an invasive diagnostic or therapeutic intervention (e.g. re-operation, needle aspiration at surgical site, gastroscopy, coloscopy, ...);
- Circulatory decompensation (related to overt bleeding leading to a systolic blood pressure [SBP] of < 90mmHg or requiring massive fluids infusion or vasopressor support to maintain SBP ≥ 90 mmHg).

A clinically relevant non-major bleeding is defined as:
- Skin hematoma, excluding wound hematoma, requiring surgical or medical intervention. Wound hematoma requiring intervention (e.g. needle aspiration) is adjudicated as a major bleeding according to the definition above (bleeding leading to an invasive diagnostic or therapeutic intervention - e.g. re-operation, needle aspiration at surgical site, gastroscopy, coloscopy, ...). In addition, an unusual wound hematoma that does not require surgical or medical intervention maybe adjudicated as a clinically relevant non-major bleeding;
- Epistaxis requiring surgical or medical treatment (i.e. packing);
- Macroscopic hematuria not mainly caused by instrumentation (i.e. difficult catheter insertion);
- Unscheduled contact/attention (of Health Care Professional) due to an overt bleeding event but not fulfilling criteria for major bleeding (i.e. prolonged bleeding after venipuncture, ...).

As used herein, the wording "semuloparin for ..." shall be understood as being equivalent to the wording "use of semuloparin for ..." or "use of semuloparin for the preparation of a medicament for use in ...".

In an embodiment of the instant invention, the standard antithrombotic treatment consists in the LMWH known under the INN (International Nonproprietary Name) enoxaparin.

In another embodiment of the instant invention, semuloparin or a pharmaceutically acceptable salt thereof is used as an antithrombotic treatment in patients undergoing major orthopaedic surgery, and said use involves an improved benefit-risk profile for the prevention of deep vein thrombosis, compared to a standard antithrombotic treatment. Deep vein thrombosis designates a blood clot in a deep vein of the lower limbs.

In another embodiment of the instant invention, said antithrombotic treatment is administered to patients undergoing knee replacement surgery, hip replacement surgery or hip fracture surgery. As it will be apparent from the detailed illustration of the invention below, said antithrombotic treatment is administered to the patient after surgery, for about 1 week.

According to the instant invention, semuloparin is administered at a 20 mg daily dose to patients with normal renal function or to patients with mild or moderate renal impairment. For patients with severe renal impairment, semuloparin is administered at a 10 mg daily dose.

According to the instant invention, the renal function of the patients is defined according to estimated creatinine clearance (CLcr) values calculated using the well-known Cockroft-Gault formula, and is classified according to the following characteristics :
- normal renal function: CLcr > 80 mL/min;
- mild renal impairment: 50 ≤ CLcr ≤ 80 mL/min;
- moderate renal impairment: 30 ≤CLcr < 50 mL/min;
- severe renal impairment: CLcr < 30 mL/min.

The treatment with semuloparin is advantageously administered once daily. As used therein, "daily" means an administration every 24 hours plus or minus 2 hours. Said treatment is advantageously administered for 7 to 10 days.

In the instant invention, the improved benefit-risk profile is clinically proven by a meta-analysis of phase III clinical trials. It shall be understood that "phase III clinical trials" refer to multicenter, randomized, double-blind studies involving large patients groups, aiming at being the definitive assessment of how effective and safe the drug is, in comparison with current standard treatment.

The invention also relates to the use of semuloparin or a pharmaceutically acceptable salt thereof for the manufacture of a medicament useful as an antithrombotic treatment in patients undergoing major orthopaedic surgery, wherein said use involves an improved benefit-risk profile compared to a standard antithrombotic treatment. The embodiments as described above also apply to said use. In particular, said use is clinically proven by a meta-analysis of phase III clinical trials.

Having now described the present invention, the same will be more clearly understood by reference to the following examples of the invention, which are included herewith for purposes of illustration only and are not intended to be limiting of the invention.

The following abbreviations shall be used:
- CIAC:: Central Independent Adjudication Committee
- CLcr:: Creatinine Clearance
- DVT:: deep vein thrombosis
- IP:: investigational product
- PE:: pulmonary embolism
- UFH:: unfractionated heparin
- LMWH:: low molecular weight heparin
- OR:: Odds Ratio
- q.d.:: *quaque die* (once daily)
- s.c.:: subcutaneously
- SRI:: severe renal impairment
- VTE:: venous thromboembolism
- 95% mid-p CI:: 95% mid-p Confidence Interval

### Example 1: Preparation of semuloparin

Semuloparin, in the form of a sodium salt, is obtained by a chemoselective depolymerization of heparin, activated through its benzyl ester derivative, by the phosphazene base 2-tert-butylimino-2-diethylamino-1,3-dimethylperhydro-1,2,3-diazaphosphorine (BEMP). The hemisynthetic pathway, allowing to recover semuloparin in the form of a sodium salt, is described in the patent application WO 2004/033503 and in Journal of Thrombosis and Haemostasis, 2009, vol. 7, 1143-1151. This procedure yields semuloparin with an average molecular weight around 2400 Da, an anti-Factor Xa activity of ∼ 160 U/mg and a residual anti-Factor IIa activity (∼ 2 U/mg).

### Example 2 : Efficacy and safety meta-analysis in orthopaedic surgery

**2.1: The SAVE-KNEE study.** A Multinational, Multicenter, Randomized, Double Blind Study comparing the Efficacy and Safety of AVE5026 with enoxaparin for the Prevention of Venous Thromboembolism in Patients Undergoing Elective Knee Replacement Surgery.

### 1 ) Study objectives

The primary objective of the study is to compare the efficacy of once daily (q.d.) subcutaneous (s.c.) injections of 20 mg AVE5026 (10 mg for SRI patients) with twice daily (b.i.d.) s.c. injections of 30 mg enoxaparin (20 mg q.d. for SRI patients) administered during 7-10 days after surgery for the prevention of venous thromboembolic events in patients undergoing elective knee replacement surgery. The secondary objectives of this study are to evaluate the safety of AVE5026 in patients undergoing elective knee replacement surgery and to document AVE5026 exposures in this population.

### 2) Study design

Patient's eligibility is determined during the screening period (within the 2 weeks prior to surgery) and is reviewed before randomization (day before surgery or day of surgery).

Randomized treatment is allocated to eligible patients, taking into account the geographical region of the patient and the estimated CLcr at screening (< or ≥ 30 mL/min).

An end of treatment visit is performed the day of last IP injection or at Day 10, whichever comes first. A bilateral venography is performed between Day 7 and Day 11. A follow-up visit is scheduled at Day 35-42.

Maximum duration of study participation is therefore 42 days, including a treatment period up to Day 7-10 and a follow-up period with a visit at Day 35-42.

### 3) Patients

A total of 1150 patients were randomized in the study.

Patients meeting the following criteria are suitable for enrolment in the study:
- Elective knee replacement surgery or a revision of at least one component of a previously implanted knee prosthesis performed ≥ 6 months prior to study entry.
- Signed written informed consent.

Patients meeting one of the following criteria are excluded from enrolment into the study:
1. Legal lower age limitations (country specific).
2. Any major orthopedic surgery in the 3 months prior to study start.
3. Elective knee surgery with polyethylene liner exchange only.
4. First step of a two-step exchange arthroplasty for infection after knee replacement.
5. Hemostasis prior to the first IP injection not established
6. Clinical signs or symptoms of DVT or PE within the last 12 months or known post phlebitic syndrome.
7. Known sensitivity to iodine or contrast dyes, and any contra-indications to the performance of venography.
8. Any treatment or procedure within 2 weeks prior to randomization, or planned during the course of the study treatment period, that could affect the incidence of VTE, such as:
   - Parenteral anticoagulants (UFH, LMWH [eg, enoxaparin, dalteparin, nadroparin], fondaparinux, bivalirudin, hirudin)
   - Oral anticoagulants (vitamin K antagonists)
   - GPllb/Illa antagonists: abciximab, eptifibatide, tirofiban
   - Thrombolytic agents
   - Dextrans
   - Intermittent pneumatic compression of the legs (IPC)
9. Known progressive malignant disease.
10. Subject unlikely to comply with protocol (eg, uncooperative attitude, inability to return for follow-up visits, inability to receive daily injection by a Health Care Professional after hospital discharge and unlikelihood of completing the study).
11. Treatment with any investigational product or investigational device in the last 30 days or 5 half lives (whichever is longer) prior to randomization.
12. Any previous exposure to AVE5026
13. Active major bleeding.
14. Thrombocytopenia associated with a positive in vitro test for anti-platelet antibody in the presence of enoxaparin sodium.
15. Known hypersensitivity to enoxaparin sodium (eg, pruritus, urticaria, anaphylactoid reactions).
16. Known hypersensitivity to heparin or pork products.
17. Conditions with increased risk of hemorrhage, such as bacterial endocarditis, congenital or acquired bleeding disorders, active ulcerative and angiodysplastic gastrointestinal disease, hemorrhagic stroke, or shortly after brain, spinal, or ophthalmological surgery.
18. End stage renal disease (estimated creatinine clearance <10 mL/min) or patient on dialysis.
19. Pregnant or breast-feeding women.
20. Women of childbearing potential not protected by highly effective contraceptive method of birth control as defined for contraception in the Informed Consent Form for the duration of the study and/or who are unwilling or unable to be tested for pregnancy.

### 4) Treatments

Sanofi-aventis supplies and manufactures the blinded treatments for this study. According to their randomized assignments, patients receive either AVE5026 or enoxaparin. Both treatments are presented as a ready-to-use 0.5 ml prefilled syringe, identical in appearance, and containing the same volume of a sterile, isotonic solution with sodium chloride 0.9 % and water for injection.

The matching placebo syringe is strictly identical in appearance, containing the same volume but without active component.

AVE5026 or its placebo are administered subcutaneously. The entire volume of the pre-filled syringe must be injected.

Investigational Product (IP) is administered in a blind manner for 7 to 10 days after surgery (Day 1 is the day of surgery). Patients receive either:
- Enoxaparin, 30 mg sc b.i.d. (20 mg q.d. for patients with SRI, defined as estimated CLcr < 30 mL/min), or
- AVE5026, 20 mg sc q.d. (10 mg q.d. for patients with SRI).

### 5) Assessment of efficacy and safety

The efficacy analysis period is defined as the period from randomization up to Day 11 (Day 1 being the day of surgery) or up to the mandatory bilateral venography, whichever comes first.

The primary efficacy population includes all randomized patients who received at least one IP injection (active or placebo), who underwent elective knee replacement surgery and with a non-missing primary efficacy endpoint.

Safety parameters include, amongst other parameters, bleedings up to 3 calendar days after last IP injection and up to Day 42. Bleedings are adjudicated by a Central Independent Adjudication Committee.

**2.2: The SAVE-HIP1 study.** A Multinational, Multicenter, Randomized, Double Blind Study comparing the Efficacy and Safety of AVE5026 with enoxaparin for the Prevention of Venous Thromboembolism in Patients Undergoing Elective Total Hip Replacement Surgery.

### 1) Study objectives

The primary objective of the study is to compare the efficacy of once daily (q.d.) subcutaneous (s.c.) injections of 20 mg AVE5026 (10 mg for patients with SRI) with q.d. s.c. injections of 40 mg enoxaparin (20 mg for patients with SRI) administered during 7-10 days after surgery for the prevention of venous thromboembolic events in patients undergoing elective hip replacement surgery. The secondary objectives of this study are to evaluate the safety of AVE5026 in patients undergoing elective total hip replacement surgery and to document AVE5026 exposures in this population.

### 2) Study design

It is proceeded in the same manner as described above (for the SAVE-KNEE study).

### 3) Patients

A total of 2326 patients were randomized in the study.

Patients meeting the following criteria are suitable for enrolment in the study:
- Elective hip replacement surgery or a revision of at least one component of a previously implanted total hip prosthesis performed ≥ 6 months prior to study entry;
- Signed written informed consent.

Patients meeting one of the following criteria are excluded from enrolment into the study:
1. Elective hip surgery with polyethylene liner exchange only.
2. First step of a two-step exchange arthroplasty for infection after hip prosthesis replacement.
3. Any of the exclusion criteria numbered as items 1, 2 and 6-20 under example 2.1 above (SAVE-KNEE study).

### 4) Treatments, assessment of efficacy and safety

It is proceeded in the same manner as described above (for the SAVE-KNEE study).

**2.3: The SAVE-HIP2 study.** A Multinational, Multicenter, Randomized, Double Blind Study comparing the Efficacy and Safety of AVE5026 with enoxaparin for the Prevention of Venous Thromboembolism in Patients Undergoing Hip Fracture Surgery.

### 1) Study objectives

The primary objective of the study is to compare the efficacy of once daily (q.d.) subcutaneous (s.c.) injections of 20 mg AVE5026 (10 mg for patients with SRI) with q.d. s.c. injections of 40 mg enoxaparin (20 mg for patients with SRI) administered for 7-10 days after surgery for the prevention of venous thromboembolic events in patients undergoing hip fracture surgery. The secondary objectives of this study are to evaluate the safety of AVE5026 in patients undergoing hip fracture surgery and to document AVE5026 exposures in this population.

### 2) Study design

It is proceeded in the same manner as described above (see SAVE-KNEE study).

### 3) Patients

A total of 1003 patients were randomized in the study.

Patients meeting the following criteria are suitable for enrolment in the study:
1. Standard surgery for fracture of the upper third of the femur, including femoral head and neck:
   - either planned within the first 36 hours* after admission to hospital, and in the 12 ± 1 hours following the pre-operative IP injection,
   - or just performed 8 ± 1 hours previously (from the time of incision closure), and provided that hemostasis has been established.
      * If time from injury to hospital admission is documented to be less than 24 hours, this 36 hours time window can be expanded but in all cases the time from injury to surgery must not exceed 60 hours.
2. Signed written informed consent.

Patients meeting one of the following criteria are excluded from enrolment into the study:
1. Estimated time of injury/fracture >24 hours before admission to hospital.
2. Time from injury/fracture to surgery >60 hours.
3. Multiple trauma affecting more than one organ system.
4. Any of the exclusion criteria numbered as items 1, 2 and 6-20 described under example 2.2 above (SAVE-KNEE study).

### 4) Treatments, assessment of efficacy and safety

It is proceeded in the same manner as described above (for the SAVE-KNEE study).

### 2.4: Meta-analysis of the SAVE-KNEE, SAVE-HIP-1 and SAVE-HIP-2 orthopaedic surgery results

A meta-analysis of the three studies described in examples 2.1, 2.2 and 2.3 above was performed using a fixed-effect logistic regression model adjusted on study. Of 4479 patients randomized, 3457 were evaluable for the primary efficacy analysis and 4436 were evaluable for the safety analysis.

The occurrence of the primary end-point of any VTE or all-cause death was significantly lower for semuloparin *versus* enoxaparin, as shown in table 1 below. Table 2 below details the components of the primary efficacy endpoint, demonstrating an improved efficacy for semuloparin, *versus* enoxaparin, in terms of prevention of DVT.

**Table 1: Metaanalysis - Any VTE or death during the efficacy analysis period**

| | Semuloparin | Enoxaparin |
|---|---|---|
| | (N=1728) | (N = 1729) |
| Any VTE or death: | | |
| n (%) | 231 (13.4%) | 305 (17.6%) |
| Comparison versus enoxaparin: | | |
| . Common OR (95% exact CI) | | 0.70 (0.58 to 0.85) |
| . p-value | | 0.0003 |

| | | |
|---|---|---|
| N: number of patients in the primary efficacy population n: number of patients showing a given event | | |

**Table 2: Metaanalysis - Components of the primary efficacy endpoint**

| | Semuloparin | Enoxaparin | OR |
|---|---|---|---|
| | | | (95% mid-p CI) |
| Any DVT | | | |
| n/N (%) | 225/1722 (13.1 %) | 301/1725 (17.4%) | 0.69 (0.57 to 0.84) |
| Any proximal DVT | | | |
| n/N (%) | 43/1912 (2.2%) | 51/1918 (2.7%) | 0.84 (0.55 to 1.26) |
| Distal DVT only | | | |
| n/N (%) | 175/1718 (10.2%) | 234/1716 (13.6%) | 0.70 (0.57 to 0.87) |
| Non-fatal PE | | | |
| n/N (%) | 1 /2211 (<0.1%) | 1/2219 (<0.1%) | 1.01 (0.06 to 16.12) |
| All cause death | | | |
| n/N (%) | 5/2211 (0.2%) | 4/2219 (0.2%) | 1.27 (0.34 to 4.75) |

| | | | |
|---|---|---|---|
| N: number of efficacy evaluable patients n: number of patients showing a given event | | | |

As shown in table 3 below, semuloparin exhibits a similar safety, in terms of clinically relevant bleedings, than enoxaparin, while a trend for improvement in the incidence of major bleedings is observed in the group treated with semuloparin, compared to the group treated with enoxaparin.

**Table 3: Meta-analysis - Patients with any treatment-emergent clinically relevant bleedings and patients with treatment-emergent major bleedings in the safety population**

| | Semuloparin | Enoxaparin |
|---|---|---|
| | (N = 2214) | (N = 2222) |
| Any clinically relevant bleedings: | | |
| n (%) | 37 (1.7%) | 39 (1.8%) |
| Comparison versus enoxaparin: | | |
| OR (95% mid-p CI) | | 0.95 (0.60 to 1.50) |
| Any major bleedings: | | |
| n (%) | 12 (0.5%) | 21 (0.9%) |
| Comparison versus enoxaparin: | | |
| OR (95% mid-p CI) | | 0.57 (0.27 to 1.16) |

| | | |
|---|---|---|
| N: number of patients in the safety population n: number of patients with bleeding | | |

These results demonstrate that semuloparin exhibits an improved efficacy compared to enoxaparin with a similar safety, leading to an improved benefit-risk profile in patients undergoing major orthopaedic surgery.

## Claims

1. Semuloparin or a pharmaceutically acceptable salt thereof for use as an antithrombotic treatment in patients undergoing major orthopaedic surgery, wherein said use involves an improved benefit-risk profile for the prevention of venous thromboembolism and death, compared to a standard antithrombotic treatment.

2. Semuloparin or a pharmaceutically acceptable salt thereof for the use according to claim 1, wherein said standard antithrombotic treatment is a treatment with enoxaparin.

3. Semuloparin or a pharmaceutically acceptable salt thereof for the use according to claim 1 or claim 2, wherein said use involves an improved benefit-risk profile for the prevention of deep vein thrombosis.

4. Semuloparin or a pharmaceutically acceptable salt thereof for the use according to any of claims 1 to 3, wherein said antithrombotic treatment is administered to patients undergoing knee replacement surgery, hip replacement surgery or hip fracture surgery.

5. Semuloparin or a pharmaceutically acceptable salt thereof for the use according to any of claims 1 to 4, wherein semuloparin is administered for 7 to 10 days.

6. Semuloparin or a pharmaceutically acceptable salt thereof for the use according to any of claims 1 to 5, wherein semuloparin is administered at a 20 mg daily dose to patients with normal renal function or to patients with mild or moderate renal impairment.

7. Semuloparin or a pharmaceutically acceptable salt thereof for the use according to any of claims 1 to 5, wherein semuloparin is administered at a 10 mg daily dose in patients with severe renal impairment.

8. Semuloparin or a pharmaceutically acceptable salt thereof for the use according to any of claims 1 to 7, wherein semuloparin is administered once daily.

9. Semuloparin or a pharmaceutically acceptable salt thereof for the use according to any of claims 1 to 8, wherein said antithrombotic treatment is a prophylactic treatment for venous thromboembolism.

10. Semuloparin or a pharmaceutically acceptable salt thereof for the use according to any of claims 1 to 9, wherein said improved benefit-risk profile is clinically proven by a meta-analysis of phase III clinical trials.
